(19) Europäisches Patentamt
European Patent Office
Office européen des brevets

(11) Veröffentlichungsnummer: **0 005 516**
**B1**

(12) **EUROPÄISCHE PATENTSCHRIFT**

(21) Anmeldenummer: **79101443.4**

(22) Anmeldetag: **11.05.79**

(51) Int. Cl.³: **A 01 N 43/58,**
**C 07 D 237/22**

# (54) Substituierte Pyridazone, Verfahren zu ihrer Herstellung und ihre Anwendung als Herbizide

(30) Priorität: **19.05.78 DE 2821809**

(43) Veröffentlichungstag der Anmeldung:
**28.11.79 Patentblatt 79/24**

(45) Bekanntmachung des Hinweises auf die Patenterteilung:
**14.01.81 Patentblatt 81/02**

(84) Benannte Vertragsstaaten:
**AT BE CH DE FR GB IT LU NL SE**

(56) Entgegenhaltungen:
**Keine**

(73) Patentinhaber: **BASF Aktiengesellschaft**
**Carl-Bosch-Strasse 38**
**D-6700 Ludwigshafen (DE)**

(72) Erfinder: **Theobald, Hans, Dr.**
**Parkstrasse 2**
**6703 Limburgerhof (DE)**
**Wuerzer, Bruno, Dr. Diplom-Landwirt**
**Wilhelm-Busch-Strasse 55**
**6703 Limburgerhof (DE)**
**Kiehs, Karl. Dr.**
**Sudetenstrasse 22**
**D-6840 Lampertheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 005 516 B1

Substituierte Pyridazone, Verfahren zu ihrer Herstellung und ihre Anwendung als Herbizide

Die vorliegende Anmeldung betrifft neue wertvolle Pyridazone-(6) mit guter herbizider Wirkung, Herbizide, die diese Verbindungen enthalten, und Verfahren zur Bekämpfung unerwünschten Pflanzenwunchses mit diesen Verbindungen.

1-Phenyl-4-dialkylamino-pyridazone und 1-Cyclohexyl-4-dialkylamino-pyridazone sind als selektive Herbizide bekannt (DBP 1 105 232, DBP 1 123 510).

Es wurde gefunden, daß Pyridazon-(6)-derivate der allgemeinen Formel

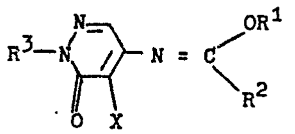

in der X Halogen (Cl, Br),
$R^1$ $C_1$—$C_6$-Alkyl, ($CH_3$, $C_2H_5$)
$R^2$ H, $C_1$—$C_6$-Alkyl ($CH_3$, $C_2H_5$) oder Haloalkyl (Cl—$CH_2$) und
$R^3$ Phenyl oder Cyclohexyl bedeutet, bei Vor- oder Nachauflaufanwendung eine beachtliche herbizide Wirkung entfalten und hierbei in selektiver Weise Kulturpflanzen schonen.

Die neuen Verbindungen werden hergestellt durch Umsetzung von Orthocarbonsäureestern (III) mit Pyridazonderivaten (II) in Gegenwart von Säuren bei Temperaturen von 80 bis 200°C. Die Umsetzung erfolgt beispielsweise nach dem folgenden Schema

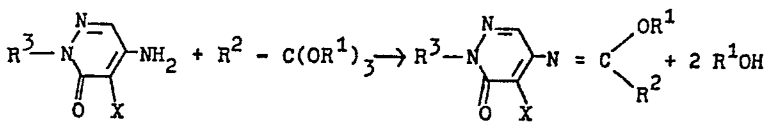

II III I

wobei X, $R^1$, $R^2$ und $R^3$ die oben genannten Bedeutungen haben.

Die bei der Umsetzung anwesenden Säuren sind beispielsweise: Phosphorsäure, Schwefelsäure, Halogenwasserstoffsäure, organische Sulfonsäuren, Halogenessigsäuren. Der bei der Umsetzung entstehende Alkohol $R^1OH$ wird aus dem Reaktionsgemisch abdestilliert. Bei der Umsetzung von II mit III zu I werden äquimolare Mengen II und III, bevorzugt aber ein Überschuß von III, eingesetzt. Als Lösungsmittel können III selbst oder alle Lösungsmittel, die nicht mit III, II oder I reagieren und einen höheren Siedepunkt haben als $R^1OH$, Verwendung finden, z.B. Benzol, Toluol, Halogenbenzole.

Beispiel 1

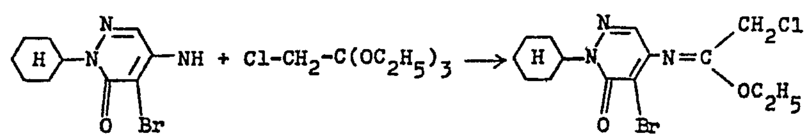

230 g 1-Cyclohexyl-5-brom-4-amino-pyridazon-(6),
1 Liter o-Chloressigsäuretriäthylester,
5 g o-Phosphorsäure
wurden gemischt, auf 130°C erhitzt und dabei freiwerdender Äthylalkohol aus dem Reaktionsgemisch abdestilliert. Es wurde anschließend 1 Stunde bei 150°C gerührt, danach abgekühlt und die Reaktionslösung mit 5%iger wäßriger $Na_2CO_3$-Lösung und mit Wasser gewaschen; nach Trocknung über $Na_2SO_4$ wurde eingeengt und der Rückstand aus Petroläther kristallisiert. Man erhält 273 g der Verbindung 1 (86% Ausbeute). Fp. 74—78°C.

| | | | | | |
|---|---|---|---|---|---|
| Ber.: | C 44,6 | H 5,1 | N 11,2 | Cl 9,44 | Br 21,2 |
| Gef.: | C 44,9 | H 5,2 | N 11,4 | Cl 8,9 | Br 21,0 |

Verwendet man anstelle von 1-Cyclohexyl-5-brom-4-amino-pyridazon-(6) das 1-Phenyl-5-brom-4-amino-pyridazon-(6) dann erhält man in entsprechender Weise die Verbindung

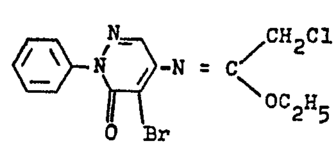

(Verb. 19)

In entsprechender Weise wurden die folgenden Verbindungen hergestellt:

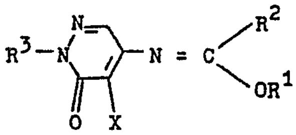

| Verbindung Nr. | X | $R^2$ | $R^1$ | $R^3$ | Fp.(°C) |
|---|---|---|---|---|---|
| 2. | Br | H | $C_2H_5$ | $C_6H_{11}$ | 95—97 |
| 3. | Br | $Ch_3$ | $CH_3$ | $C_6H_{11}$ | 100—101 |
| 4. | Br | $CH_3$ | $C_2H_5$ | $C_6H_{11}$ | 60-MHz-NMR-Spektrum in $CDCl_3$ ($\delta$-Werte) 1,24 (3H); 1,84 (3H); 1,3-1,7 (10H); 4,02 (2H); 4,71 (1H); 7,19 (1H) |
| 5. | Br | $C_2H_5$ | $C_2H_5$ | $C_6H_{11}$ | 60-MHz-Spektrum in $CDCl_3$ ($\delta$ -Werte) 1,01 (3H); 1,24 (3H); 1,3-1,82 (10H); 1,97 (2H); 4.13 (2H); 4,74 (1H); 7,22 (1H). |
| 6. | Br | H | $CH_3$ | $C_6H_{11}$ | 124—125 |
| 7. | Br | $CH_2Cl$ | $CH_3$ | $C_6H_{11}$ | 122—125 |
| 8. | Cl | $CH_3$ | $C_2H_5$ | $C_6H_{11}$ | 89— 91 |
| 9. | Cl | $C_2H_5$ | $C_2H_5$ | $C_6H_{11}$ | 270 MHz-NMR-Spektrum in $CDCl_3$ ($\delta$ -Werte) 1,1 (3H); 1,28 (3H); 1,15-1,95 (10H); 2,2 (2H); 4,3 (2H); 4,95 (1H); 7,5 (1H) |
| 10. | Cl | H | $CH_3$ | $C_6H_{11}$ | 93 |
| 11. | Cl | $CH_2Cl$ | $C_2H_5$ | $C_6H_{11}$ | 73—74 |
| 12. | Cl | H | $C_2H_5$ | $C_6H_{11}$ | 105—115 |
| 13. | Cl | H | $C_2H_5$ | $C_6H_5$ | 128—130 |
| 14. | Br | H | $CH_3$ | $C_6H_5$ | 85—88 |
| 15. | Br | H | $C_2H_5$ | $C_6H_5$ | 113—114 |
| 16. | Br | $CH_3$ | $CH_3$ | $C_6H_5$ | 98—100 |
| 17. | Br | $CH_3$ | $C_2H_5$ | $C_6H_5$ | 57—60 |
| 18. | Br | $C_2H_5$ | $C_2H_5$ | $C_6H_5$ | 78—80 |
| 19. | Br | $CH_2Cl$ | $C_2H_5$ | $C_6H_5$ | 80—81 |
| 20. | Cl | $CH_3$ | $C_2H_5$ | $C_6H_5$ | |
| 21. | Cl | H | $CH_3$ | $C_6H_5$ | 102 |

| Verbindung Nr. | X | $R^2$ | $R^1$ | $R^3$ | Fp.(°C) |
|---|---|---|---|---|---|
| 22. | Cl | $CH_2Cl$ | $C_2H_5$ | $C_6H_5$ | 84—85 |
| 23. | Cl | $C_2H_5$ | $C_2H_5$ | $C_6H_5$ | 74—77 |
| 24. | Br | $CH_2Cl$ | $CH_3$ | $C_6H_5$ | 80 |
| 25. | Cl | $CH_2Cl$ | $CH_3$ | $C_6H_5$ | 91—93 |

Der Einfluß verschiedener Vertreter der neuen Verbindungen auf das Wachstum von Pflanzen wird in den folgenden Versuchen demonstriert.

I. Gewächshausversuche Als Kulturgefäße dienten Plastikblumentöpfe mit 300 $cm^3$ Inhalt gefüllt mit lehmigem Sand mit etwa 1,5% Humus. Die Samen der Testpflanzen entsprechend Tabelle 1 wurden nach Arten getrennt flach eingesät. Bei Cyperus esculentus nahm man vorgekeimte Knollen. Unmittelbar danach erfolgte bei Vorauflaufbehandlung das Aufbringen der Wirkstoffe auf die Erdoberfläche. Sie wurden hierbei in Wasser als Verteilungsmittel suspendiert oder emulgiert und mittels fein verteilender Düsen gespritzt. Nach dem Aufbringen der Mittel wurden die Gefäße leicht beregnet, um Keimung und Wachstum in Gang zu bringen und die chemischen Mittel zu aktivieren. Danach deckte man die Gefäße mit durchsichtigen Plastikhauben ab, bis die Pflanzen angewachsen waren. Diese Abdeckung bewirkte ein gleichmäßiges Keimen der Testpflanzen, sofern dies nicht durch die Chemikalien beeinträchtigt wurde.

Zum Zwecke der Nachauflaufbehandlung zog man die Pflanzen je nach Wuchsform in den Versuchsgefäßen bis zu einer Höhe von 3 bis 10 cm an und behandelte sie danach. Eine Abdeckung unterlieb. Die Aufstellung der Töpfe erfolgte im Gewächshaus, wobei für wärmeliebende Arten wärmere Bereiche (25 bis 40°C) und für solche gemäßigter Klimate 15 bis 30°C bevorzugt wurden. Die Versuchsperiode erstreckte sich über 4 bis 6 Wochen. Während dieser Zeit wurden die Pflanzen gepflegt, und ihre Reaktion auf die einzelnen Behandlungen wurde ausgewertet. Die folgenden Tabellen enthalten die Prüfsubstanzen, die jeweiligen Aufwandmengen berechnet in kg/ha Aktivsubstanz und die Testpflanzenarten. Bewertet wird nach einer Skala von 0 bis 100. Dabei bedeutet 0 keine Schädigung oder normaler Auflauf und 100 kein Aufgang der Pflanzen bzw. völlige Zerstörung zumindest der oberirdischen Sproßteile.

II. Freilandversuche

Es handelt sich dabei um Kleinparzellenversuche auf Standorten mit lehmigem Sand mit pH 5—6 und 1—1,5% Humusgehalt. Es werden Vorauflaufanwendungen beschrieben, welche jeweils unmittelbar bis spätestens 3 Tage nach der Saat erfolgten. Die Kulturpflanzen wurden jeweils in Reihen gesät. Die Unkrautflora verschiedenster Artenzusammensetzung war natürlich vorkommend. Die Substanzen wurden in Wasser als Träger- und Verteilermedium emulgiert oder suspendiert, mit Hilfe einer motorgetriebenen, auf einen Geräteträger montierten Parzellenspritz ausgebracht. Bei Fehlen natürlicher Niederschläge wurde künstlich beregnet, um Keimung und Wachstum von Nutzpflanzen und Unkräutern zu gewährleisten. Alle Versuche liefen über mehrere Wochen oder Monate. In diesem Zeitraum wurde die Bewertung nach der Skala 0 bis 100 vorgenommen.

*Ergebnis*

Die Tabellen 2, 3 und 4 zeigen die Ergebnisse, welche bei der Bekämpfung grasartiger und breitblättriger unerwünschter Pflanzen mit den neuen Verbindungen erzielt wurden. Zuckerrüben und Sonnenblumen erwiesen sich als Beispiele für Nutzpflanzen, die von den Wirkstoffen nicht geschädigt wurden.

In ihrer herbiziden Wirkung sind die neuen Verbindungen so stark, daß sie bei höheren Dosierungen jeglichen Wuchs krautiger Pflanzen total vernichten. Hier eröffnen sich Anwendungsgebiete wie die Beseitigung von unerwünschtem Pflanzenwuchs in Strauch- und Baumkulturen sowie auf Wegen, Plätzen, Industrie- und Gleisanlagen.

In Tabellen wird die Anwendung bei Vor- und Nachauflaufbehandlung dokumentiert. Selbstverständlich können neben der Oberflächenspritzung (surface application) die Mittel in den Boden eingearbeitet werden. Dabei kann es sich bei Kulturpflanzenbeständen um eine Einarbeitung vor der Saat, nach der Saat oder zwischen bereits etablierten Nutzpflanzen handeln. Es können aber auch Ausbringungstechniken angewandt werden, bei welchen die Mittel mit Hilfe der Spritzgeräte so gespritzt werden, daß die Blätter empfindlicher Kulturpflanzen nach Möglichkeit nicht getroffen werden, während sie auf die darunterliegende Bodenfläche oder dort wachsende unerwünschte Pflanzen gelangen (post directed, lay-by).

TABELLE 1 – Liste der Pflanzennamen

| Botanischer Name | Abkürz. in Tabellen | Deutscher Name | Englischer Name |
|---|---|---|---|
| Alopecurus myosuroides | Alopec. myos. | Ackerfuchsschwanz | slender foxtail |
| Beta vulgaris spp. alt. | Z.-Rüben | Zuckerrüben | sugarbeets |
| Chenopodium album | Chenop. album | Weißer Gänsefuß | lambsquarters (goosefoot) |
| Cyperus difformis | Cyper. diff. | | smallflower umbrellaplant |
| Cyperus ferax | Cyper. ferax | | |
| Datura stramonium | Dat. stram. | Gemeiner Steckapfel | Jimsonweed |
| Digitaria sanguinalis | Digita. sang. | Blutfingerhirse | large crabgrass |
| Echinochloa crus galli | Echino. c. g. | Hühnerhirse | barnyardgrass |
| Euphorbia geniculata | Euphorb. genic. | Südamerikanische Wolfsmilchart | Southamerican member of the spurge family |
| Helianthus annuus | Helianth. ann. | Sonnenblume | sunflowers |
| Matricaria / Anthemis spp. | Matric. / Anthem. spp. | Kamillearten | chamomile |
| Mercurialis annua | Mercur. annua | Einjähriges Bingelkraut | annual mercury |
| Polygonum spp. | Polygon. spp. | Knötericharten | |
| Sesbania exaltata | Sesbania exalt. | Turibaum | hemp sesbania (coffeeweed) |
| Sinapis alba | Sinapis alba | Weißer Senf | white mustard |
| Sinapis arvensis | Sinapis arv. | Ackersenf | yellow charlock |
| Solanum nigrum | Solanum nigrum | Schwarzer Nachschatten | black nightshade |
| Stellaria media | Stellaria media | Vogelsternmiere | chickweed |

TABELLE 2 – Selektive herbizide Wirkung von Pyridazonderivaten bei Vorauflaufanwendung im Freiland

| Wirkstoff Nr. | kg/ha | Testpflanzen und % Schädigung | | | | | | |
|---|---|---|---|---|---|---|---|---|
| | | Z.-Rüben | Alopec. myos. | Chenop. album | Matric. / Anthem. spp. | Polygon spp. | Sinapis arv. | Stellaria media |
| 1-Phenyl-4-dimethylamino-5-chlor-pyridazon-(6) | 1,5 | 0 | 8 | 7 | 20 | 0 | 8 | 7 |
| bekannt | 2,0 | – | 29 | – | 28 | – | – | 10 |
| 1-Phenyl-4-dimethylamino-5-brom-pyridazon-(6) bekannt | 1,5 | – | 10 | 10 | 5 | 0 | 15 | 15 |
| 1-Phenyl-4-diäthylamino-5-chlor-pyridazon-(6) bekannt | 1,5 | 0 | 5 | 7 | 7 | 2 | 5 | 7 |
| 1-Phenyl-4-diäthylamino-5-brom-pyridazon-(6) bekannt | 1,5 | 0 | 15 | 20 | 5 | 0 | 10 | 10 |

Fortsetzung von Tabelle 2

| Wirkstoff Nr. | kg/ha | Testpflanzen und % Schädigung<br>Z.-Rüben | Alopec. myos. | Chenop. album | Matric. / Anthem. spp. | Polygon. spp. | Sinapis arv. | Stellaria media |
|---|---|---|---|---|---|---|---|---|
| 13 | 1,5 | 0 | – | 48 | 80 | – | – | – |
| | 2,0 | 0 | 63 | 90 | 77 | 88 | 84 | 97 |
| 14 | 1,5 | 2 | 40 | 97 | 100 | 55 | 53 | 80 |
| | 2,0 | 6 | 71 | 92 | 100 | 82 | 84 | 82 |
| 15 | 1,5 | 6 | 49 | 81 | 100 | 85 | 55 | 93 |
| | 2,0 | 13 | 73 | 91 | 100 | 94 | 92 | 99 |
| 16 | 1,0 | 0 | 70 | 70 | – | – | 100 | 90 |
| | 2,0 | 0 | 95 | 98 | 78 | – | 100 | 87 |
| 17 | 1,5 | 0 | 56 | 72 | 98 | 70 | – | 64 |
| | 2,0 | 0 | 72 | 86 | 99 | 84 | 80 | 80 |
| 18 | 1,0 | 0 | – | 51 | 90 | 52 | 26 | 23 |
| | 2,0 | 0,4 | – | 78 | 98 | 66 | 51 | 53 |
| 19 | 1,5 | 0 | 54 | 94 | 100 | 93 | – | 82 |
| | 2,0 | 4 | 74 | 98 | 100 | 95 | – | 92 |

0 = keine Schädigung, 100 = Pflanzen nicht aufgelaufen oder abgestorben.

TABELLE 3 – Selektive herbizide Wirkung von Pyridazonderivaten bei Vorauflaufanwendung im Gewächshaus

| Wirkstoff | kg/ha | Testpflanzen und % Schädigung<br>Helianth. ann. | Cyper. ferax | Digita. sang. | Echino.. c.g. | Euphorb. genic. | Sesbania exalt. | Sinapis alba | Solanum nigrum | Stellaria media |
|---|---|---|---|---|---|---|---|---|---|---|
| 10 | 1,0 | 0 | 90 | 100 | 100 | 45 | 98 | 60 | 98 | 30 |
| | 2,0 | 8 | 90 | 100 | 100 | 100 | 100 | 100 | 100 | 100 |
| 6 | 1,0 | 10 | 100 | 100 | 75 | 100 | 98 | 100 | 100 | 100 |
| | 2,0 | 15 | 100 | 100 | 80 | 100 | 100 | – | 100 | 100 |
| 12 | 1,0 | 0 | 100 | 40 | 40 | 40 | 100 | 100 | 100 | 100 |
| | 2,0 | 5 | 100 | 80 | 70 | 100 | 100 | 100 | 100 | 100 |
| 2 | 1,0 | 10 | 95 | 95 | 100 | 100 | 100 | 100 | 100 | 100 |
| | 2,0 | 18 | 95 | 95 | 100 | 100 | 100 | 100 | 100 | 100 |
| 3 | 1,0 | 0 | 100 | 60 | 100 | 80 | – | 98 | 65 | 100 |
| | 2,0 | 0 | 100 | 100 | 100 | 100 | – | 98 | 97 | 100 |
| 4 | 1,0 | 0 | 100 | 100 | 70 | 100 | 98 | 100 | 100 | 100 |
| | 2,0 | 0 | 100 | 100 | 100 | 100 | 100 | 100 | 100 | 100 |

Fortsetzung von TABELLE 3

| Wirkstoff Nr. | kg/ha | Testpflanzen und % Schädigung<br>Helianth. ann. | Cyper. ferax | Digita. sang. | Echino. c.g. | Euphorb. genic. | Sesbania exalt. | Sinapis alba | Solanum nigrum | Stellaria media |
|---|---|---|---|---|---|---|---|---|---|---|
| 11 | 1,0<br>2,0 | 5<br>5 | 95<br>95 | –<br>90 | 40<br>60 | 40<br>100 | 98<br>100 | 80<br>100 | 70<br>75 | –<br>100 |
| 1 | 1,0<br>2,0 | 2,5<br>2,5 | 100<br>100 | 100<br>100 | 100<br>100 | 70<br>100 | 100<br>100 | –<br>100 | 90<br>100 | –<br>100 |
| 9 | 1,0<br>2,0 | –<br>– | 90<br>95 | 80<br>100 | 90<br>100 | –<br>– | 80<br>100 | 70<br>70 | 72<br>99 | –<br>100 |
| 5 | 1,0<br>2,0 | 5<br>15 | 95<br>95 | 100<br>100 | –<br>100 | 100<br>100 | 85<br>100 | 100<br>100 | 95<br>100 | 100<br>100 |

0 = keine Schädigung; 100 = Pflanzen nicht aufgelaufen oder abgestorben.

TABELLE 4 – Selektive herbizide Wirkung weiterer Verbindungen bei Nachauflaufanwendung im Gewächshaus

| Wirkstoff Nr. | kg/ha | Testpflanzen und % Schädigung | | | | | | | | |
|---|---|---|---|---|---|---|---|---|---|---|
| | | Helianth. ann. | Cyper. diff. | Dat. stram. | Digita sang. | Echino. c.g. | Mercur. annua | Sinapis alba | Solanum nigrum | Stellaria media |
| 6 | 0,5 | 0 | 60 | 100 | – | 95 | 80 | 100 | 100 | 100 |
| | 1,5 | 10 | 80 | 100 | 100 | 98 | 100 | 100 | 100 | 100 |
| 3 | 0,5 | 0 | 92 | 100 | 60 | 80 | 80 | 100 | 75 | 68 |
| | 1,0 | 0 | 92 | 100 | 80 | 93 | 80 | 100 | 100 | 100 |
| 2 | 0,5 | 0 | 95 | 100 | 100 | 98 | 100 | 100 | 85 | 100 |
| | 1,0 | 8 | 95 | 100 | 100 | 100 | 100 | 100 | 100 | 100 |

In Anbetracht der Vielseitigkeit der Applikationsmethoden können die erfindungsgemäßen Mittel oder diese enthaltende Mischungen außer bei den in den Tabellen aufgeführten Nutzpflanzen noch in einer weiteren großen Zahl von Kulturpflanzen zur Beseitigung oder Niederhaltung unerwünschten Pflanzenwuchses eingesetzt werden. Die Aufwandmengen können dabei von 0,1 bis 15 kg/ha und mehr je nach dem Bekämpfungsobjekt schwanken.

Im einzelnen seien folgende Nutzpflanzen genannt:

| Botanischer Name | Deutscher Name | Englischer Name |
|---|---|---|
| Allium cepa | Küchenzwiebel | onions |
| Ananas comosus | Ananas | pineapple |
| Arachis hypogaea | Erdnuß | peanuts (groundnuts) |
| Asparagus officinalis | Spargel | asparagus |
| Avena sativa | Hafer | oats |
| Beta vulgaris spp. altissima | Zuckerrübe | sugarbeets |
| Beta vulgaris spp. rapa | Futterrübe | fooder beets |
| Beta vulgaris spp. esculenta | Rote Rübe | table beets, red beets |
| Brassica napus var. napus | Raps | rape |
| Brassica napus var. napobrassica | Kohlrübe | |
| Brassica napus var. rapa | Weiße Rübe | turnips |
| Brassica rapas var. silvestris | Rübsen | |
| Camellia sinensis | Teestrauch | tea plants |
| Carthamus tinctorius | Saflor – Färberdistel | safflower |
| Carya illinoinensis | Pekannußbaum | pecan trees |
| Citrus limon | Zitrone | lemon |
| Citrus maxima | Pampelmuse | grapefruits |
| Citrus reticulata | Mandarine | |
| Citrus sinensis | Apfelsine, Orange | orange trees |
| Coffea arabica (Coffea canephora, Coffea liberica) | Kaffee | coffee plants |
| Cucumis melo | Melone | melons |
| Cucumis sativus | Gurke | cucumber |
| Cynodon dactylon | Bermudagrass | Bermudagrass in turfs and lawns |
| Daucus carota | Möhre | carrots |
| Elais guineensis | Ölpalme | oil palms |
| Fragaria vesca | Erdbeere | strawberries |
| Glycine max | Sojabohne | soybeans |

| Botanischer Name | Deutscher Name | Englischer Name |
|---|---|---|
| Gossypium hirsutum<br>(Gossypium arboreum<br>Gossypium herbaceum<br>Gossypium vitifolium) | Baumwolle | cotton |
| Helianthus annuus | Sonnenblume | sunflowers |
| Helianthus tuberosus | Topinambur | |
| Hevea brasiliensis | Parakautschukbaum | rubber plants |
| Hordeum vulgare | Gerste | barley |
| Humulus lupulus | Hopfen | hop |
| Ipomoea batatas | Süßkartoffeln | sweet potato |
| Juglans regia | Walnußbaum | walnut trees |
| Lactuca sativa | Kopfsalat | lettuce |
| Lens culinaris | Linse | lentils |
| Linum usitatissimum | Faserlein | flax |
| Lycopersicon lycopersicum | Tomate | tomato |
| Malus spp. | Apfel | apple trees |
| Manihot esculenta | Maniok | cassava |
| Medicago sativa | Luzerne | alfalfa (lucerne) |
| Mentha piperita | Pfefferminze | peppermint |
| Musa spp. | Obst- und Mehlbanane | banana plants |
| Nicothiana tabacum<br>(N. rustica) | Tabak | tobacco |
| Olea europaea | Ölbaum | olive trees |
| Oryza sativa | Reis | rice |
| Panicum miliaceum | Rispenhirse | |
| Phaseolus lunatus | Mondbohne | limabeans |
| Phaseolus mungo | Urdbohne | mungbeans |
| Phaseolus vulgaris | Buschbohnen | snapbeans, green beans,<br>dry beans |
| Pennisetum glaucum | Perl- oder Rohrkolbenhirse | |
| Petroselinum crispum<br>spp. tuberosum | Wurzelpetersilie | parsley |
| Picea abies | Rotfichte | Norway spruce |
| Abies alba | Weißtanne | fire |

| Botanischer Name | Deutscher Name | Englischer Name |
|---|---|---|
| Pinus spp. | Kiefer | pine trees |
| Pisum sativum | Gartenerbse | English peas |
| Prunus avium | Süßkirsche | Cherry trees |
| Prunus domestica | Pflaume | plum trees |
| Prunus dulcis | Mandelbaum | almond trees |
| Prunus persica | Pfirsisch | peach trees |
| Pyrus communis | Birne | pear trees |
| Ribes sylvestre | Rote Johannisbeere | red currants |
| Ribes uva-crispa | Stachelbeere | |
| Ricinus communis | Rizinus | |
| Saccharum officinarum | Zuckerrohr | sugar cane |
| Secale cereale | Roggen | rye |
| Sesamum indicum | Sesam | Sesami |
| Solanumtuberosum | Kartoffel | Irish potatoes |
| Sorghum bicolor (s. vulgare) | Mohrenhirse | grain sorghum |
| Sorghum dochna | Zuckerhirse | |
| Spinacia oleracea | Spinat | spinach |
| Theobroma cacao | Kakaobaum | cacao plants |
| Trifolium pratense | Rotklee | red clover |
| Triticum aestivum | Weizen | wheat |
| Vaccinium corymbosum | Kulturheidelbeere | blueberry |
| Vaccinium vitis-idaea | Preißelbeere | cranberry |
| Vicia faba | Pferdebohnen | tick beans |
| Vigna sinensis (V. unguiculata) | Kuhbohne | cow peas |
| Vitis vinifera | Weinrebe | grapes |
| Zea mays | Mais | Indian corn, sweet corn, maize |

Die neuen Pyridazone können mit zahlreichen Vertretern anderer herbizider oder wachstumsregulierender Wirkstoffgruppen gemischt und gemeinsam ausgebracht werden. Solche Kombinationen dienen zur Verbreiterung des Wirkungsspektrums und erzielen zuweilen synergistische Effekte.

Außerdem ist es nützlich, die neuen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralstofflösungen, welche zur Behebung von Ernährungs- oder Spurenelementmängeln eingesetzt werden. Ebenso bietet sich die Mischung mit Stickstoff-, Phosphat-oder Kali enthaltenden Mineraldünger an.

Die Anwendung erfolgt z.B. in Form von direkt versprühbaren Lösungen, Pulvern, Suspensionen auch hochprozentige wäßrige, ölige oder sonstige Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte, Naphthaline oder deren Derivate, z.B. Methanol, Äthanol, Propanol, Butanol, Chloroform, Tetrachlorkohlenstoff, Cyclohexanol, Cyclohexanon, Chlorbenzol, Isophoron usw., stark polare Lösungsmittel, z.B. Dimethylformamid, Dimethylsulfoxid, N-Methylpyrrolidon, Wasser usw. in Betracht.

Wäßrige Anwendungsformen können aus Emulsionskonzentrationen, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

An oberflächenaktiven Stoffen sind zu nennen:

Alkali-, Erdalkali-, Ammoniumsalze von Ligninsulfonsäure, Naphthalinsulfonsäuren, Phenolsulfonsäure, Alkylarylsulfonate, Alkylsulfate, Alkylsulfonate, Alkali- und Erdalkalisalze der Dibutylnaphthalinsulfonsäure, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyäthylenoctylphenoläther, äthoxyliertes Isooctylphenol, Octylphenol, Nonylphenol, Alkylphenolpolyglykoläther, Tributylphenyl-polyglykoläther, Alkylarylpolyätheralkohole, Isotridecylalkohol, Fettalkoholäthylenoxid-Kondensate, äthoxyliertes Rizinusöl, Polyoxyäthylenalkyläther, äthoxyliertes Polyoxypropylen, Laurylalkoholpolyglykolätheracetal, Sorbitester, Lignin, Sulfitablaugen und Methylcellulose.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Kalzium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getriedemehle, Baumrinden-, Holz- und Nußschalenmehl, Cellulosepulver und andere feste Trägerstoffe.

Die Formulierungen enthalten zwischen 0,1 und 95 Gewichtsprozent Wirkstoffe, vorzugsweise zwischen 0,5 und 90 Gewichtsprozent.

Zu den Mischungen oder Einzelwirkstoffen können Öle verschiedenen Typs, Netz- oder Haftmittel, Herbizide, Fungizide, Nematozide, Insektizide, Bakterizide, Spurenelemente, Düngemittel, Antischaummittel (z.B. Silikone), Wachstumsregulatoren, Antidotmittel oder andere herbizid wirksame Verbindungen zugesetzt werden. Die Zumischung dieser Mittel zu den erfindungsgemäßen Herbiziden kann im Gewichtsverhältnis 1 : 10 bis 10 : 1 erfolgen. Das gleiche gilt für Öle, Netz- oder Haftmittel, Fungizide, Nematozide, Insektizide, Bakterizide, Antidotmittel und Wachstumsregulatoren.

Beispiel 2

Man vermischt 90 Gewichtsteile der Verbindung 1 mit 10 Gewichtsteilen N-Methyl-$\alpha$-pyrrolidon und erhält eine Lösung, die zur Anwendung in Form kleinster Tropfen geeignet ist.

Beispiel 3

20 Gewichtsteile der Verbindung 2 werden in einer Mischung gelöst, die aus 80 Gewichtsteilen Xylol, 10 Gewichtsteilen des Anlagerungsproduktes von 8 bis 10 Mol Äthylenoxid an 1 Mol Ölsäure-N-mono-äthanolamid, 5 Gewichtsteilen Calciumsalz der Dodecylbenzosulfonsäure und 5 Gewichtsteilen des Anlagerungsproduktes von 40 Mol Äthylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

Beispiel 4

20 Gewichtsteile der Verbindung 2 werden in einer Mischung gelöst, die aus 40 Gewichtsteilen

Cyclohexanon, 30 Gewichtsteilen Isobutanol, 20 Gewichtsteilen des Anlagerungsproduktes von 7 Mol Äthylenoxid an 1 Mol Isooctylphenol and 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,22 Gewichtsprozent des Wirkstoffs enthält.

Beispiel 5

20 Gewichtsteile der Verbindung 2 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,22 Gewichtsprozent des Wirkstoffs enthält.

Beispiel 6

20 Gewichtsteile des Wirkstoffs 2 werden mit 3 Gewichtsteilen des Natriumsalzes der Diisobutylnaphthalin-$\alpha$-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

Beispiel 7

3 Gewichtsteile der Verbindung 1 werden mit 97 Gewichtsteilen feinteiligem Kaolin innig vermischt. Man erhält auf diese Weise ein Stäubemittel, das 3 Gewichtsprozent des Wirkstoffs enthält.

Beispiel 8

30 Gewichtsteile der Verbindung 2 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel 9

40 Gewichtsteile des Wirkstoffs 1 werden mit 10 Teilen Natriumsalz eines Phenolsulfonsäure-Harnstoff-Formaldehyd-Kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gewichtsprozent Wirkstoff enthält.

Beispiel 10

20 Teile des Wirkstoffs 2 werden mit 2 Teilen Calciumsalz der Dodecylbenzolsulfonsäure, 8 Teilen Fettalkohol-polyglykoläther, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

**Patentansprüche für die Vertragsstaatem: BE CH DE FR GB IT LU NL SE**

1. Pyridazon-(6)-derivat der Formel

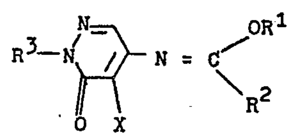

I

in der X Halogen,
$R^1$ $C_1$—$C_6$-Alkyl,
$R^2$ H, $C_1$—$C_6$-Alkyl oder Haloalkyl und
$R^3$ Phenyl oder Cyclohexyl bedeutet.

2. Verbindung der Formel

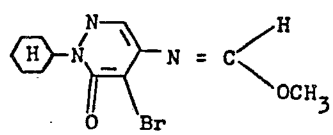

3. Verbindung der Formel

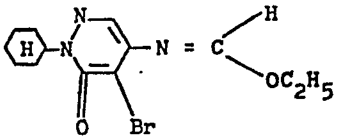

4. Verbindung der Formel

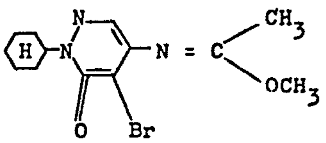

5. Herbizid, enthaltend eine Verbindung gemäß Anspruch 1, 2, 3 oder 4.

6. Herbizid, enthaltend einen festen oder flüssigen Trägerstoff und eine Verbindung gemäß Anspruch 1, 2, 3 oder 4.

7. Verfahren zur Herstellung eines Herbizids, *dadurch gekennzeichnet,* daß man einen festen oder flüssigen Trägerstoff vermischt mit einer Verbindung gemäß Anspruch 1.

8. Verfahren zur Bekämpfung unerwünschten Pflanzenwuchses, *dadurch gekennzeichnet,* daß man die Pflanzen oder den Boden behandelt mit einer herbizid wirksamen Menge einer Verbindung gemäß Anspruch 1.

9. Verfahren zur Herstellung von Verbindungen gemäß Anspruch 1, *dadurch gekennzeichnet,* daß man einen Orthocarbonsäureester der Formel $R^2—C(OR^1)_3$, in der $R^1$ und $R^2$ die oben angeführten Bedeutungen haben, umsetzt mit einer Verbindung der Formel

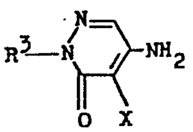

in der $R^3$ und X die oben angeführten Bedeutungen haben, in Gegenwart einer Säure bei einer Temperatur von 80 bis 200°C.

**Claims for the Contracting states: BE CH DE FR GB IT LU NL SE**

1. A pyridaz-6-one derivative of the formula

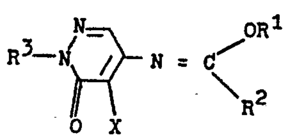

where X denotes halogen, $R^1$ denotes $C_1—C_6$-alkyl,
$R^2$ denotes H, $C_1—C_6$-alkyl or haloalkyl, and
$R_3$ denotes phenyl or cyclohexyl.

2. A compound of the formula

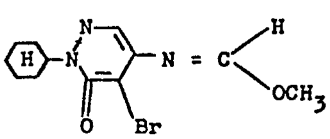

3. A compound of the formula

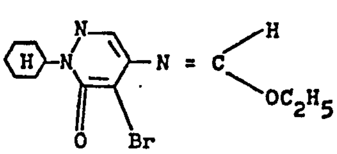

6. Herbicide contenant un support solide ou liquide et un composé selon l'une des revendications 1 à 4.

7. Procédé de préparation d'un herbicide caractérisé par le fait que l'on mélange un support solide ou liquide avec un composé selon la revendication 1.

8. Procédé pour lutter contre la croissance de plantes adventices, caractérisé par le fait que l'on traite les plantes ou le sol avec un composé selon la revendication 1 en quantité procurant un effet herbicide.

9. Procédé pour préparer des composés selon la revendication 1, caractérisé par le fait que l'on fait réagir un ester d'acide orthocarboxylique de formule $R^2—C(OR^1)_3$, dans laquelle $R^1$ et $R^2$ ont les significations indiquées ci-dessus avec un composé de formule

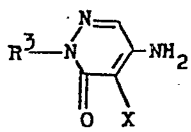

dans laquelle $R^3$ et X ont les significations indiquées, en présence d'un acide à une température de 80 à 200°C.

**Patentansprüche für dem Vertragsstaat: AT**

1. Herbizid, enthaltend ein Pyridazon-(6)-derivat der Formel

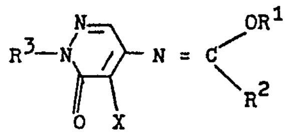

in der X Halogen,
$R^1$ $C_1—C_6$-Alkyl,
$R^2$ H, $C_1—C_6$-Alkyl oder Haloalkyl und
$R^3$ Phenyl oder Cyclohexyl bedeutet.

2. Herbizid, enthaltend eine Verbindung der Formel

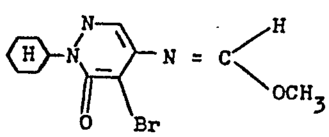

3. Herbizid, enthaltend eine Verbindung der Formel

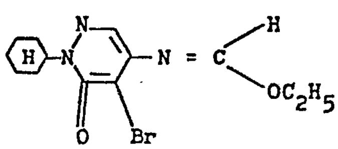

4. Herbizid, enthaltend eine Verbindung der Formel

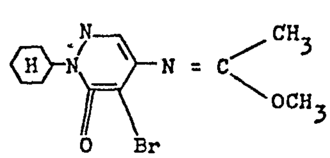

5. Verfahren zur Bekämpfung unerwünschten Pflanzenwuchses, *dadurch gekennzeichnet,* daß man die Pflanzen oder den Boden behandelt mit einem Herbizid gemäß Anspruch 1.

6. Verfahren zur Herstellung von Verbindungen für ein Herbizid gemäß Anspruch 1, *dadurch gekennzeichnet,* daß man einen Orthocarbonsäureester der Formel $R^2—C(OR^1)_3$, in der $R^1$ und $R^2$ die oben angeführten Bedeutungen haben, umsetzt mit einer Verbindung der Formel

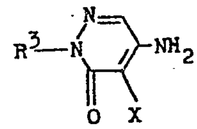

in der $R^3$ und X die oben angeführten Bedeutungen haben, in Gegenwart einer Säure bei einer Temperatur von 80 bis 200°C.

**Claims for the Contracting state: AT**

1. A herbicide containing a pyridaz-6-one derivative of the formula

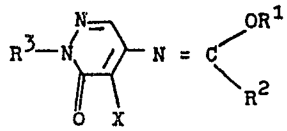

where X denotes halogen, $R^1$ denotes $C_1$—$C_6$-alkyl,
$R^2$ denotes H, $C_1$—$C_6$-alkyl or haloalkyl, and
$R^3$ denotes phenyl or cyclohexyl.

2. A herbicide containing a compound of the formula

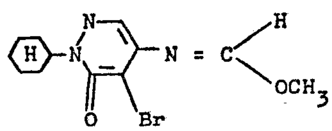

3. A herbicide containing a compound of the formula

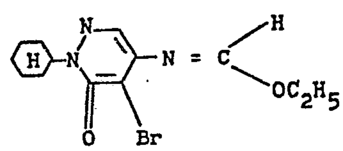

4. A herbicide containing a compound of the formula

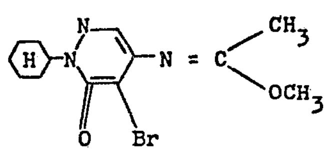

5. A process for combating unwanted plant growth, *characterised in that* the plants or the soil are treated with a herbicide as claimed in claim 1.

6. A process for the preparation of compounds for a herbicide as claimed in claim 1, *characterised in that* an o-carboxylic acid ester of the formula $R^2$—$C(OR^1)_3$, where $R^1$ and $R^2$ have the above meanings, is reacted with a compound of the formula

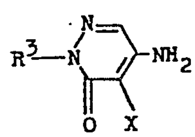

where $R_3$ and X have the above meanings, in the presence of an acid at from 80° to 200°C.

3. Verbindung der Formel

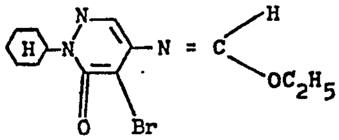

4. Verbindung der Formel

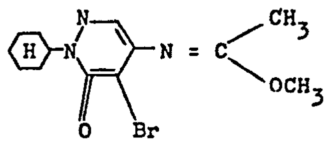

5. Herbizid, enthaltend eine Verbindung gemäß Anspruch 1, 2, 3 oder 4.

6. Herbizid, enthaltend einen festen oder flüssigen Trägerstoff und eine Verbindung gemäß Anspruch 1, 2, 3 oder 4.

7. Verfahren zur Herstellung eines Herbizids, *dadurch gekennzeichnet,* daß man einen festen oder flüssigen Trägerstoff vermischt mit einer Verbindung gemäß Anspruch 1.

8. Verfahren zur Bekämpfung unerwünschten Pflanzenwuchses, *dadurch gekennzeichnet,* daß man die Pflanzen oder den Boden behandelt mit einer herbizid wirksamen Menge einer Verbindung gemäß Anspruch 1.

9. Verfahren zur Herstellung von Verbindungen gemäß Anspruch 1, *dadurch gekennzeichnet,* daß man einen Orthocarbonsäureester der Formel $R^2—C(OR^1)_3$, in der $R^1$ und $R^2$ die oben angeführten Bedeutungen haben, umsetzt mit einer Verbindung der Formel

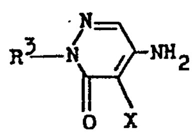

in der $R^3$ und X die oben angeführten Bedeutungen haben, in Gegenwart einer Säure bei einer Temperatur von 80 bis 200°C.

**Claims for the Contracting states: BE CH DE FR GB IT LU NL SE**

1. A pyridaz-6-one derivative of the formula

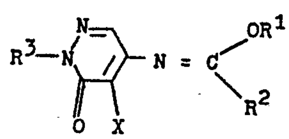

where X denotes halogen, $R^1$ denotes $C_1—C_6$-alkyl,
$R^2$ denotes H, $C_1—C_6$-alkyl or haloalkyl, and
$R_3$ denotes phenyl or cyclohexyl.

2. A compound of the formula

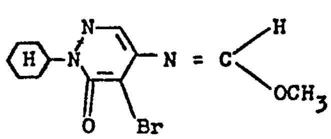

3. A compound of the formula

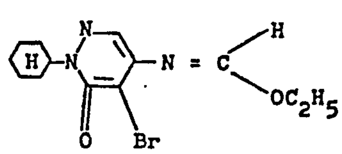

4. A compound of the formula

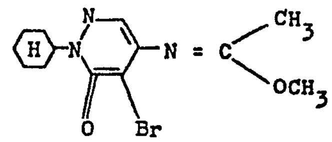

5. A herbicide containing a compound as claimed in claim 1, 2, 3 or 4.

6. A herbicide containing a solid or liquid carrier material and a compound as claimed in claim 1, 2, 3 or 4.

7. A process for the preparation of a herbicide, *characterised in that* a solid or liquid carrier material is mixed with a compound as claimed in claim 1.

8. A process for combating unwanted plant growth, *characterised in that* the plants or the soil are treated with a herbicidally effective amount of a compound as claimed in claim 1.

9. A process for the preparation of compounds for a herbicide as claimed in claim 1, *characterised in that* an o-carboxylic acid ester of the formula $R^2—C(OR^1)_3$, where $R^1$ and $R^2$ have the above meanings, is reacted with a compound of the formula

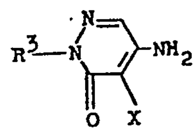

where $R^3$ and X have the above meanings, in the presence of an acid at from 80° to 200°C.

**Revendications pour les Etats contractants: BE CH DE FR GB IT LU NL SE**

1. Dérivé de pyridazone-(6)- de formule

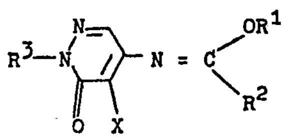

I

dans laquelle X est un halogène,
$R^1$ un reste alkyle en $C_1$ à $C_6$
$R^2$ de l'hydrogène, un reste alkyl ou halogène-alkyle en $C_1$ à $C_6$, et
$R^3$ un reste phényle ou cyclohexyle.

2. Composé de formule

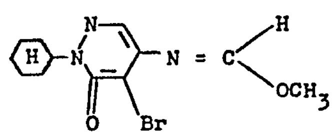

3. Composé de formule

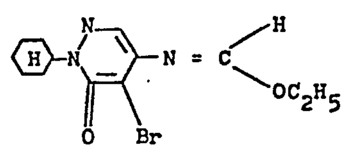

4. Composé de formule

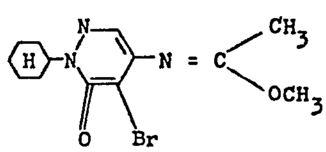

5. Herbicide contenant un composé selon l'une des revendications 1 à 4.

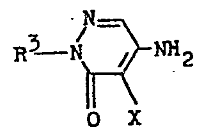

in der $R^3$ und X die oben angeführten Bedeutungen haben, in Gegenwart einer Säure bei einer Temperatur von 80 bis 200°C.

**Claims for the Contracting state: AT**

1. A herbicide containing a pyridaz-6-one derivative of the formula

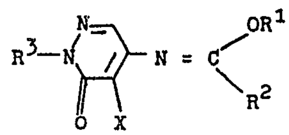

where X denotes halogen, $R^1$ denotes $C_1$—$C_6$-alkyl,
$R^2$ denotes H, $C_1$—$C_6$-alkyl or haloalkyl, and
$R^3$ denotes phenyl or cyclohexyl.

2. A herbicide containing a compound of the formula

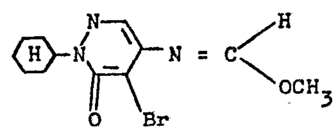

3. A herbicide containing a compound of the formula

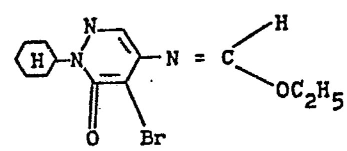

4. A herbicide containing a compound of the formula

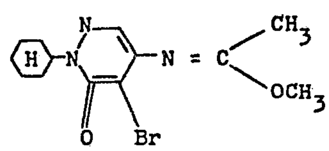

5. A process for combating unwanted plant growth, *characterised in that* the plants or the soil are treated with a herbicide as claimed in claim 1.

6. A process for the preparation of compounds for a herbicide as claimed in claim 1, *characterised in that* an o-carboxylic acid ester of the formula $R^2$—$C(OR^1)_3$, where $R^1$ and $R^2$ have the above meanings, is reacted with a compound of the formula

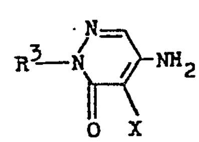

where $R_3$ and X have the above meanings, in the presence of an acid at from 80° to 200°C.

4. A compound of the formula

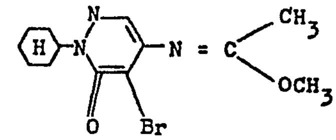

5. A herbicide containing a compound as claimed in claim 1, 2, 3 or 4.

6. A herbicide containing a solid or liquid carrier material and a compound as claimed in claim 1, 2, 3 or 4.

7. A process for the preparation of a herbicide, *characterised in that* a solid or liquid carrier material is mixed with a compound as claimed in claim 1.

8. A process for combating unwanted plant growth, *characterised in that* the plants or the soil are treated with a herbicidally effective amount of a compound as claimed in claim 1.

9. A process for the preparation of compounds for a herbicide as claimed in claim 1, *characterised in that* an o-carboxylic acid ester of the formula $R^2$—$C(OR^1)_3$, where $R^1$ and $R^2$ have the above meanings, is reacted with a compound of the formula

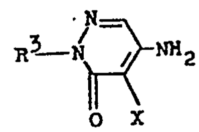

where $R^3$ and X have the above meanings, in the presence of an acid at from 80° to 200°C.

**Revendications pour les Etats contractants: BE CH DE FR GB IT LU NL SE**

1. Dérivé de pyridazone-(6)- de formule

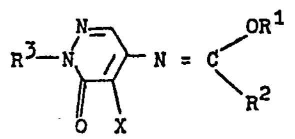

I

dans laquelle X est un halogène,
$R^1$ un reste alkyle en $C_1$ à $C_6$
$R^2$ de l'hydrogène, un reste alkyl ou halogène-alkyle en $C_1$ à $C_6$, et
$R^3$ un reste phényle ou cyclohexyle.

2. Composé de formule

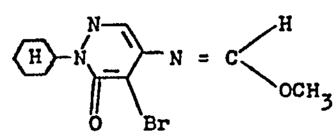

3. Composé de formule

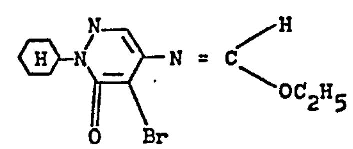

4. Composé de formule

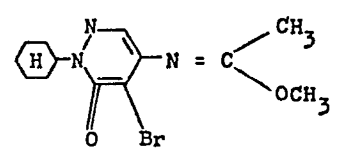

5. Herbicide contenant un composé selon l'une des revendications 1 à 4.

6. Herbicide contenant un support solide ou liquide et un composé selon l'une des revendications 1 à 4.

7. Procédé de préparation d'un herbicide caractérisé par le fait que l'on mélange un support solide ou liquide avec un composé selon la revendication 1.

8. Procédé pour lutter contre la croissance de plantes adventices, caractérisé par le fait que l'on traite les plantes ou le sol avec un composé selon la revendication 1 en quantité procurant un effet herbicide.

9. Procédé pour préparer des composés selon la revendication 1, caractérisé par le fait que l'on fait réagir un ester d'acide orthocarboxylique de formule $R^2—C(OR^1)_3$, dans laquelle $R^1$ et $R^2$ ont les significations indiquées ci-dessus avec un composé de formule

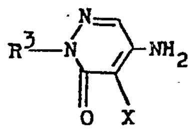

dans laquelle $R^3$ et X ont les significations indiquées, en présence d'un acide à une température de 80 à 200°C.

**Patentansprüche für dem Vertragsstaat: AT**

1. Herbizid, enthaltend ein Pyridazon-(6)-derivat der Formel

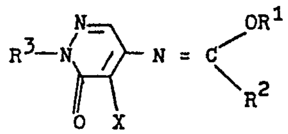

in der X Halogen,
$R^1$ $C_1—C_6$-Alkyl,
$R^2$ H, $C_1—C_6$-Alkyl oder Haloalkyl und
$R^3$ Phenyl oder Cyclohexyl bedeutet.

2. Herbizid, enthaltend eine Verbindung der Formel

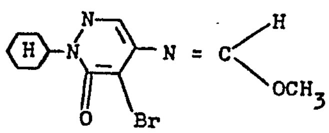

3. Herbizid, enthaltend eine Verbindung der Formel

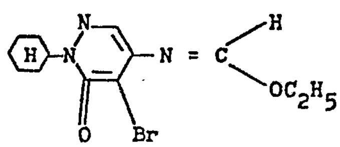

4. Herbizid, enthaltend eine Verbindung der Formel

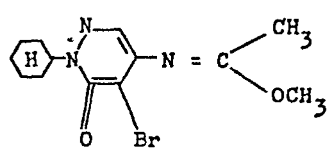

5. Verfahren zur Bekämpfung unerwünschten Pflanzenwuchses, *dadurch gekennzeichnet,* daß man die Pflanzen oder den Boden behandelt mit einem Herbizid gemäß Anspruch 1.

6. Verfahren zur Herstellung von Verbindungen für ein Herbizid gemäß Anspruch 1, *dadurch gekennzeichnet,* daß man einen Orthocarbonsäureester der Formel $R^2—C(OR^1)_3$, in der $R^1$ und $R^2$ die oben angeführten Bedeutungen haben, umsetzt mit einer Verbindung der Formel

**Revendications pour l'Etat contractant: AT**

1. Herbicide, contenant un dérivé de pyridazone-(6)- de formule

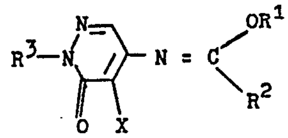

dans laquelle X est un halogène,
$R^1$ un reste alkyle en $C_1$ à $C_6$
$R^2$ de l'hydrogène, un reste alkyle ou halogène-alkyle en $C_1$ à $C_6$, et
$R^3$ un reste phényle ou cyclohexyle.

2. Herbicide, contenant un composé de formule

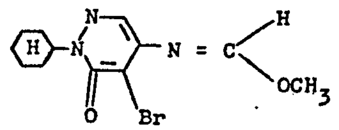

3. Herbicide, contenant un composé de formule

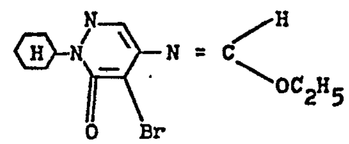

4. Herbicide, contenant un composé de formule

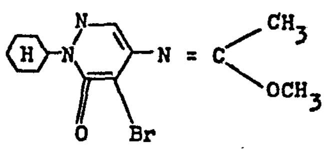

5. Procédé pour lutter contre la croissance de plantes adventices, caractérisé par le fait que l'on traite les plantes ou le sol avec un herbicide selon la revendication 1.

6. Procédé pour préparer des composés pour un herbicide selon la revendication 1, caractérisé par le fait que l'on fait réagir un ester d'acide orthocarboxylique de formule $R^2—C(OR^1)_3$, dans laquelle $R^1$ et $R^2$ ont les significations indiquées ci-dessus, avec un composé de formule

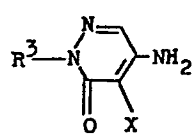

dans laquelle $R^3$ et X ont les significations indiquées ci-dessus, en présence d'un acide à une température de 80 à 200°C.